# EUROPEAN PATENT APPLICATION

(11) **EP 2 700 719 A1**
(43) Date of publication of application: **26.02.2014**
(21) Application number: 12774868.9
(22) Date of filing: 13.04.2012
(51) Int. Cl.: C12Q 1/68

(54) **METHOD AND PRIMERS FOR DETECTION OF miRNA, AND APPLICATION THEREOF**

(30) Priority: 20.04.2011 CN 201110101438
(71) Applicant: Shenzhen University, Shenzhen, Guangdong 518060 (CN)
(72) Inventor: GOU, Deming, Shenzhen Guangdong 518000 (CN); KANG, Kang, Shenzhen Guangdong 518000 (CN)
(74) Representative: Chaillot, Geneviève
(86) International application number: PCT/CN2012/073976
(87) International publication number: WO 2012/142924

(57) **Abstract**

A method and primer for quantitative assay of microRNAs (miRNAs) and application of the same are provided. In this method, miRNAs are subjected to polyadenylation and subsequent reverse transcription with miRNA-specific S-Oligo(dT) RT primers. The miRNA is then assayed in a real-time PCR quantitative assay using a miRNA-specific forward primer, a universal reverse primer and a universal probe. The methods in the present invention have improved sensitivity, specificity, and efficiency in miRNA quantification assay, providing a simple, high-throughput, and cost-effective tool applicable for the early diagnosis and prognosis of many severe diseases such as malignancy.

## Description

### FIELD

The present invention relates to a method and primer for a quantitative assay of microRNA, and application of the method and primer in early diagnosis and prognosis of severe diseases such as malignancy.

### BACKGROUND

MicroRNAs (miRNAs) are non-coding single-stranded RNAs of ∼22-nucleotides in length that widely exist in eukaryotes including animals, plants and nematodes. MiRNAs are capable of regulating gene expression through binding to the 3'UTR of their target mRNAs, and promote mRNA degradation and/or translational inhibition, thereby reducing the protein synthesis of target gene. MiRNAs play multiple regulatory roles in various biological processes, including embryo development, cell differentiation, proliferation, apoptosis, cellular stress response, immunoregulation and development of many diseases. Many studies have reported that dyregulation of miRNA is tightly associated with the development of malignancy, suggesting miRNAs as potential biomarkers for use in the early diagnosis of diseases, and as targets for therapeutics. It is of great importance for a rapid, sensitive and accurate method for quantitative assay of miRNAs in order to apply the miRNAs in the early diagnosis of severe diseases such as malignancy.

Quantitative assay of miRNAs has faced great difficulties and challenges due to the following reasons: (1) mature miRNAs are very small RNAs without poly(A) tail; (2) many miRNAs are highly conserved in sequence (in some cases there is only one-nucleotide difference); and (3) miRNAs generally express in a relatively low level. Nevertheless, scientists have developed several methods for miRNA assay, which can generally be classified into two categories: hybridization-based methods and polymerase chain reaction PCR based methods.

Hybridization-based methods for miRNA assay are direct assay methods that usually neglect the amplification of RNA samples, mainly including Northern blot, microarray, and bead-based flow cytometry. The basic principle of these technologies is to hybridize probes and RNA samples and then perform the signal detection. Among these methods, Northern blot is a classic method for RNA assay which is usually used to evaluate reliability of other methods. However, Northern bolt method requires a relatively large amount of RNA samples and is time-consuming and laborious in operation, making it unsuitable for high-throughput analysis. The microarray technology is capable of high-throughput analysis of miRNA, i.e. multiple miRNAs can be assayed at the same time on a single chip. However, the microarray technology produces low specificity results, has poor reproducibility and leads to a high experiment cost. The bead-based flow cytometry appoach can achieve a higher specificity by positioning the probe on the bead and disposing the bead in a liquid to facilitate capturing miRNA sequences. However, the bead-based flow cytometry approach requires special flow cytometers and beads which lead to a high experiment cost and therefore hinders the wide use of this technology.

On the contrary, using quantitative real-time PCR (qRT-PCR) to analyze miRNA expression is currently the most common means in miRNA research requires less RNA input for miRNA expression analysis. Generally, the results obtained using non-qRT-PCR methods all need to be further confirmed using the qRT-PCR approach. At present, there are two qRT-PCR methods for miRNA expression analysis: the poly(A) method (Shi, R.(2005) Biotechnique, 39, 519-525) and the stem-loop method (Chen, C.(2005) Nucleic Acids Res, 33, 1-9). The poly(A) method uses a poly(A) tail added to the 3' end of each mature miRNA done by poly(A) polymerase. The tailed miRNAs are then subjected to reverse transcription (RT) using a universal RT primer containing 2 to 3 degenerate nucleotides at 3' end followed by an oligo(dT) and universal reverse primer sequence. The synthesized cDNA is amplified with specific forward and universal reverse primers. This method provides a simple and cost-effective method for miRNA assay. However, as all polyadenylated RNAs are possibly recognized by the oligo(dT) RT primer, this method may reduce the specificity and sensitivity.

The stem-loop method uses an elaborately designed stem-loop RT primer that contains six specific bases at its 3' end that are complementary to the 3'end of specific mature miRNA for reverse transcription of miRNAs. The primer for reverse transcription has a stem-loop at its 5' end, which enhances the affinity of miRNA/DNA and avoids annealing of primer and pri- or pre-miRNA. Therefore, the stem-loop method generally provides a higher specificity than the poly(A) method. However, because of the use of a sequence-specific probe, the stem-loop method is less cost-effective especially in high-throughput miRNA profiling.

What is needed, therefore, is an improved method for miRNA assay.

### SUMMARY

Accordingly, the present invention is directed to a method and primer for a miRNA assay and application of the same which can overcome one or more disadvantages described above.

The present invention provides an S-Oligo(dT) primer for quantitative assay of miRNA that is composed of four segments: in the 5' to 3' direction, a universal reverse primer sequence for PCR, a universal probe binding sequence, and an oligo(dT) sequence, followed by several specific bases that are complementary to the 3' end of a target miRNA.

The S-Oligo(dT) primer for quantitative assay of miRNA is composed of four segments: in the 5' to 3' direction, 14 to 20 nucleotides of a universal reverse primer sequence for PCR, 14 to 20 nucleotides of a universal probe binding sequence, and 8 to 30 nucleotides of an oligo(dT) sequence, followed by 3 to 8 specific nucleotides that are complementary to the 3' end of a target miRNA.

The S-Oligo(dT) primer for quantitative assay of miRNA is composed of four segments: in the 5' to 3' direction, 16 nucleotides of a universal reverse primer sequence for PCR, 17 nucleotides of a universal probe binding sequence, and 11 nucleotides of an oligo(dT) sequence, followed by 6 specific nucleotides that are complementary to the 3' end of a particular miRNA.

The present invention provides a method for quantitative assay of a specific miRNA using the primer described above. The S-Oligo(dT) method includes the following steps:
S100. Design of S-Oligo(dT) Primer and miRNA Specific Forward Primer: an S-Oligo(dT) primer and a specific forward primer for the miRNA to be assayed are designed according to the sequence information of the target miRNA;
S200. Total RNA Extraction: cell lysis and total RNA extraction are performed, and the purity, integrity and concentration of the RNA sample are measured;
S300. RNA Polyadenylation: the total RNA is polyadenylated with poly(A) polymerase to obtain a RNA-PolyA;
S400. First Strand cDNA Synthesis: using the RNA-PolyA as a template, the miRNA is then reverse transcribed into cDNA using an S-Oligo(dT) primer;
S500. miRNA Real-time PCR Quantitative Assay: using the cDNA of the miRNA as a template, a real-time PCR quantitative assay is performed using a specific forward primer and a universal reverse primer, and a data analysis is then performed on the PCR result.

In the above method for quantitative assay of miRNA, the miRNA specific forward primer is a miRNA specific sequence without 3 to 8 nucleotides at the 3' end of the miRNA, and the miRNA universal reverse primer is a universal primer sequence having 14 to 20 nucleotides in length derived from the S-Oligo(dT) primer sequence.

In the above method for quantitative assay of miRNA, the assay of the real-time PCR of step S500 can be performed using either a fluorescent dye method or probe method. The probe method uses a universal probe having 14 to 20 nucleotides in length which is derived from the S-Oligo(dT) primer sequence.

The present invention also provides a method for quantitative assay of different miRNAs using the primer described above, including the following steps:
S100. Design of S-Oligo(dT) Primer and miRNA Specific Forward Primer: different S-Oligo(dT) primers and different specific forward primers for different miRNAs to be assayed are designed according to the sequence information of the different target miRNAs;
S200. Total RNA Extraction: cell lysis and total RNA extraction are performed, and the purity, integrity and concentration of the RNA samples are measured;
S300. RNA Polyadenylation: the total RNA is polyadenylated with poly(A) polymerase to obtain a RNA-PolyA;
S400. First Strand cDNA Synthesis: using the RNA-PolyA as a template, the miRNAs are then reverse transcribed into cDNAs using a combination of the different S-Oligo(dT) primers;
S500. miRNA Real-time PCR Quantitative Assay: using the cDNAs of the miRNAs as templates, real-time PCR quantitative assays are performed using respective ones of the specific forward primers and the same universal reverse primer, and a data analysis is then performed on the PCR result.

In the above method for quantitative assay of miRNA, the miRNA specific forward primer is a miRNA specific sequence without 3 to 8 nucleotides at the 3' end of the miRNA, and the miRNA universal reverse primer is a universal primer sequence having 14 to 20 nucleotides in length derived from the S-Oligo(dT) primer sequence.

In the above method for quantitative assay of miRNA, the assay of the real-time PCR of step S500 can be performed using either a fluorescent dye method or probe method. The probe method uses a universal probe having 14 to 20 nucleotides in length which is derived from the S-Oligo(dT) primer sequence.

The present invention also provides an application of the above method for quantitative assay of miRNA wherein the above method is applied in the early diagnosis and prognosis of many severe diseases such as malignancy.

The methods in the present invention have improved sensitivity, specificity, and efficiency in miRNA quantification assay, providing a simple, high-throughput, and cost-effective tool applicable in the early diagnosis and prognosis of many severe diseases such as malignancy.

### BRIEF DESCRIPTION OF THE DRAWINGS

FIG.1 is a schematic representation of an S-Oligo(dT) method for miRNA quantitative assay.
FIG. 2 shows an amplification plot of hsa-miR-21 from Hela cell over six orders of magnitude using the S-Oligo(dT) method.
FIG.3 shows a standard curve of amplification of hsa-miR-21 from Hela cell using the S-Oligo(dT) method.
FIG.4 illustrates comparison of the sensitivity of different methods in the assay of hsa-miR-21 from Hela cell.

### DESCRIPTION OF THE EMBODIMENTS

The present invention provides methods and primers for quantitative assay of miRNAs, and application of the same. Before any independent embodiments of the invention are explained in detail, it is to be understood that the invention is not limited in its application to the details of construction and the arrangement of components set forth in the following description or illustrated in the following drawings. The invention is capable of other embodiments and of being practiced or of being carried out in various ways.

The present invention provides an S-Oligo(dT) primer for quantitative assay of miRNA that is composed of four segments: in the 5' to 3' direction, a universal reverse primer sequence for PCR, a universal probe binding sequence, and an oligo(dT) sequence, followed by several specific bases that are complementary to the 3' end of a target miRNA.

The S-Oligo(dT) primer for quantitative assay of miRNA is composed of four segments: in the 5' to 3' direction, 14 to 20 nucleotides of a universal reverse primer sequence for PCR, 14 to 20 nucleotides of a universal probe binding sequence, and 8 to 30 nucleotides of an oligo(dT) sequence, followed by 3 to 8 specific nucleotides that are complementary to the 3' end of a target miRNA.

The present invention further provides a novel S-Oligo(dT) method for quantitative assay of miRNA. In this S-Oligo(dT) method, one S-Oligo(dT) primer is used to reverse transcribe one specific miRNA, or multiple different S-Oligo(dT) primers are mixed to reverse multiple different miRNA samples at the same time. Individual miRNA is then amplified with a miRNA specific forward primer and a miRNA universal reverse primer in a real-time PCR reaction. The miRNA specific forward primer is a miRNA specific sequence without 3 to 8 nucleotides at the 3' end of the miRNA, and the miRNA universal reverse primer is a universal primer sequence having 14 to 20 nucleotides in length derived from the S-Oligo(dT) primer sequence. The assay of the real-time PCR product can be performed using either a fluorescent dye method or probe method. The probe method uses a universal probe having 14 to 20 nucleotides in length which is derived from the S-Oligo(dT) primer sequence.

The S-Oligo(dT) primer used in the examples below is composed of four segments: in the 5' to 3' direction, 16 nucleotides of a universal reverse primer sequence for PCR, 17 nucleotides of a universal probe binding sequence, and 11 nucleotides of an oligo(dT) sequence, followed by 6 specific nucleotides that are complementary to the 3' end of a particular miRNA. The miRNA specific forward primer is a miRNA specific sequence without 6 nucleotides at the 3' end of the miRNA, and the miRNA universal reverse primer is a universal primer sequence having 16 nucleotides in length derived from the S-Oligo(dT) primer sequence. The universal probe has 17 nucleotides derived from the S-Oligo(dT) primer sequence.

The S-Oligo(dT) method in the present invention provide a tool applicable in the early diagnosis and prognosis of many severe diseases such as malignancy.

The S-Oligo(dT) method includes the following steps:
S100. Design of S-Oligo(dT) Primer and miRNA Specific Forward Primer: an S-Oligo(dT) primer and a specific forward primer for the miRNA to be assayed are designed according to the sequence information of the target miRNA;
S200. Total RNA Extraction: cell lysis and total RNA extraction are performed, and the purity, integrity and concentration of the RNA sample are measured;
S300. RNA Polyadenylation: the total RNA is polyadenylated with poly(A) polymerase to obtain a RNA-PolyA;
S400. First Strand cDNA Synthesis: using the RNA-PolyA as a template, the miRNA is then reverse transcribed into cDNA using an S-Oligo(dT) primer;
S500. miRNA Real-time PCR Quantitative Assay: using the cDNA of the miRNA as a template, a real-time PCR quantitative assay is performed using a specific forward primer and a universal reverse primer, and a data analysis is then performed on the PCR result.

There are several advantages in using this S-Oligo(dT) method described above.

First, this method greatly improves the specificity in miRNA quantitative assay. There are 17 nucleotides (11 dTs and 6 specific bases) at the 3' end of each S-Poly(T) RT primer that can anneal to the polyadenylated target miRNA, providing a much higher anchoring strength than that of the poly(A)-based methods (only the Oligo(dT)) or stem-loop methods (only 6 specific bases). In addition, only those miRNAs having a "6 specific bases - 11 dTs" sequence can match with the S-Oligo(dT) primer. Therefore, the S-Oligo(dT) method can well discriminate the mature miRNA from pri-miRNA and pre-miRNA.

Second, the novel S-Oligo(dT) method has an improved sensitivity. The limit of detection (LOD) is 1 pg total RNA in amplification of miRNA. This method shows a broader linear amplification range which covers six orders of magnitude (from 1pg to 10ng total RNA). The correlation coefficient R is greater than 0.99. The S-Oligo(dT) assay method shows a minimum of 6-10 fold increase in sensitivity in comparison with the stem-loop or poly(A)-based methods.

Third, the S-Oligo(dT) method provide simple and high-throughput analysis of miRNA expression. The S-Oligo(dT) method can mix the reverse transcription primers of different miRNA for cDNA synthesis of multiple miRNAs. This not only can effectively reduce error caused by sample adding, but it also enables high-throughput analysis.

Finally, the S-Oligo(dT) method is much cost-effective. The S-Oligo(dT) method uses a universal probe which greatly reduce assay cost and hence is more suitable for wide use.

### Example 1 Quantitative Assay Of hsa-miR-21 From Hela Cell

### (1) Materials

### 1. Materials:

Hela cells were in vitro cultured.

### 2. Reagents:

RNAiso plug (TakaRa), PolyA Polymerase (EPICENTRE), PrimeScript RT reagent Kit (TaKaRa), SYBR Premix Ex Taq (Perfect Real Time) (TaKaRa), Premix Ex Taq (Perfect Real Time) (TaKaRa).

### 3. Primers and Probes

| Primers/probes | Sequence (5'-3') | Detail |
|---|---|---|
| miR-21U | GCCCGCTAGCTTATCAGACTGATG | Forward primer for hsa-miR-21 |
| miR21-SRTP | GTGCAGGGTCCGAGGTCAGAGCCACC | RT primer for hsa-miR-21 |
| | TGGGCAATTTTTTTTTTTTCAACA | |
| 3UnivP | GTGCAGGGTCCGAGGT | Universal reverse primer |
| TaqMan UnivP-1 | TTGCCCAGG | Universal probe 1 |
| TaqMan UnivP-2 | TGGCTCTG | Universal probe 2 |
| TaqMan UnivP-3 | TTGCCCAGGTGGCTCTG | Universal probe 3 |

**4. Instruments**

GeneQuant pro RNA/DNA calculator, ABI PCR instrument (2720), ABI real-time PCR instrument (PRISM 7300).

### (2) Method

### 1. RNA Extraction

Total RNA from cultured Hela cells is isolated using the RNAiso Plus (TaKaRa) according to the manufacturer's protocol. The OD260/280 and concentration of RNA are determined using a spectrophotometer. The integrity of total RNA purified is checked by denaturing agarose gel electrophoresis.

### 2. Polyadenylation

For the S-Oligo(dT) method, the total RNA from cultured cells is polyadenylated with Poly(A) Polymerase Tailing Kit.

| | | |
|---|---|---|
| | RNA (0.5 µg/µl) | 2 |
| 10 × Reaction Buffer (µl) | | 1 |
| | 10 mM ATP (µl) | 1 |
| Poly(A) Polymerase (4U/µl) | | 0.5 |
| RNase-Free Water (µl) | | 5.5 |
| | Total volume (µl) | 10 |

After adding the sample, it is incubated at 37°C for 30 minutes and placed on ice.

### 3. First Strand cDNA Synthesis

MiRNA is reverse transcribed using PrimeScript RT reagent Kit and sample is added as follows:

| | | |
|---|---|---|
| RNA-PolyA (1 pg-100 ng/µl) (µl) | | 2 |
| 0.5 µM mR21-SRTP primer (µl) | | 1 |
| | H₂O (µl) | 3 |
| | Total vol (µl) | 6 |

After adding the sample, it is followed by enzyme inactivation at 65°C for 5 min and is then quickly placed on ice for 2 minutes. The following reagents are added:

| | | |
|---|---|---|
| 5 × PrimeScript Buffer (µl) | | 2 |
| PrimeScript RT Enzyme (µl) | | 0.5 |
| | H₂O (µl) | 1.5 |
| | Total vol (µl) | 4 |

The reaction is then incubated at 42°C for 15 min, and then terminated by heating at 85°C for 5 min and placed on ice. The RT product is added with 34 µl deionized water and evenly mixed.

### 4. Real-time PCR assay of Hsa-miR-21 miRNA

The Has-miR-21 miRNA is quantitative assayed using Premix Ex Taq™ (Perfect Real Time). The sample is added as follows.

| | |
|---|---|
| Premix Ex Taq (2 ×) (µl) | 10 |
| 10 µM miR-21U (µl) | 0.4 |
| 10 µM 3UnivP (µl) | 0.4 |
| 6 µM TaqMan UnivP * | 0.4 |
| hsa-miR-21 cDNA (µl) | 8.8 |
| Total vol (µl) | 20 |

The universal probe can be any one of TaqMan UnivP-1, 2, 3. TaqMan UnivP-1, 2 are LNA-modified probe primers. The TaqMan UnivP-3 is used in this example.

The real-time PCR reaction is carried out on ABI PRISM 7300 thermal cycler at 95°C for 30 seconds, followed by 40 cycles of 95°C for 5 seconds and 60°C for 31 seconds.

In this example, the universal probe is used for detection of PCR product, which has 17 nucleotides derived from the S-Oligo(dT) primer sequence. In another embodiment, the SYBR fluorescent dye method can also be used to detect PCR product, i.e. the TaqMan universal probe is replaced by SYBR Green I dye. When using the SYBR Green I dye, a dissociation curve analysis may be performed to evaluate the specificity of the PCR product.

### (3) Results

### 1. The quantitative assay of hsa-miR-21 using the S-Oligo(dT) method

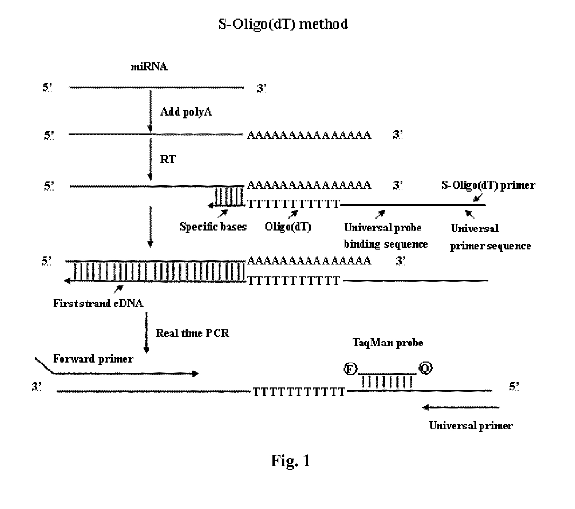

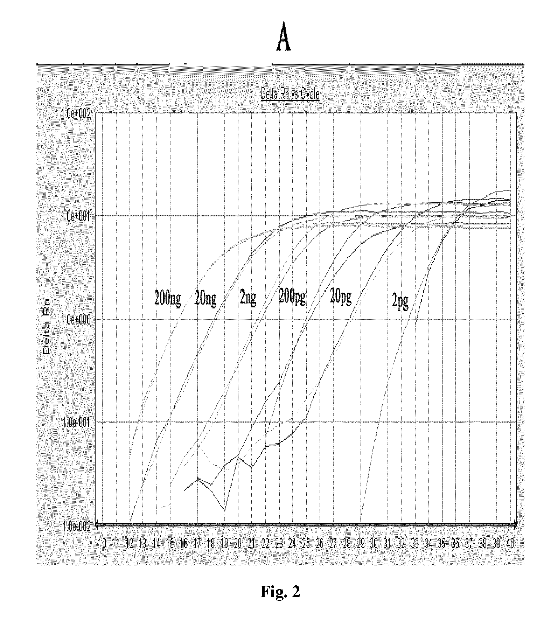

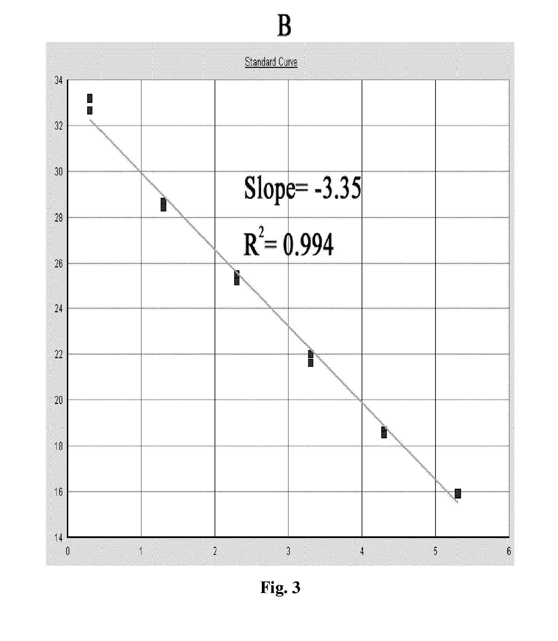

The purified total RNA from Hela cells show a good integrity by denaturing agarose gel electrophoresis. The ratio of the readings at 260 nm and 280 nm (OD_{260/280}) is 1.9, indicating a good purity of total RNA. FIG.1 shows the schematic representation of the S-Oligo(dT) method for has-miR-21 miRNA quantification. The result shows a significant amplification of hsa-miR-21 with RNA inputs ranging from 1 pg to 100 ng (Fig. 2) and an excellent linearity between the total RNA input and the threshold cycle (Ct) value (Fig. 3).

### 2. Comparison of the sensitivity of different real-time RT-PCR methods

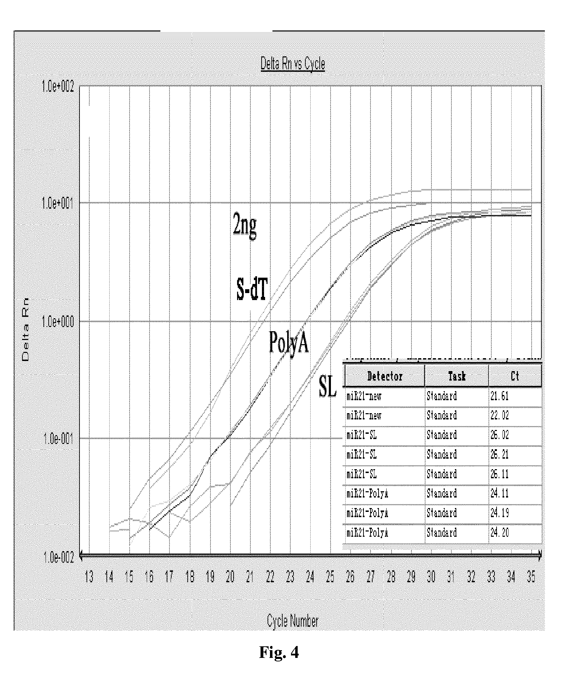

Experiments are carried out which compare the sensitivity of three different real-time RT-PCR methods including S-Oligo(dT), poly(A) and stem-loop methods to amplify the hsa-mr-21. The quantification is performed using the three real-time RT-PCR methods with 2ng total RNA as the template. The results are shown in Fig. 4. The S-dT curve represents the result of the S-Oligo(dT) method, the Poly(A) curve represents the result of the poly(A) method, and the SL curve represents the result of the stem-loop method. As can be seen from Fig. 4, the S-Oligo(dT) method produces an averaged Ct value of 21.8, while the poly(A) and stem-loop methods produce averaged Ct values of 24.2 and 26.1, respectively, demonstrating that the S-Oligo(dT) method is much more sensitive than the other two methods.

It will be apparent to those skilled in the art that various modifications and variations can be made to the disclosed structure without departing from the scope or spirit of the invention. In view of the foregoing, it is intended that the present invention cover modifications and variations of this invention provided they fall within the scope of the following claims and their equivalents.

## Claims

1. An S-Oligo(dT) primer for quantitative assay of miRNA that is composed of four segments: in the 5' to 3' direction, a universal reverse primer sequence for PCR, a universal probe binding sequence, and an oligo(dT) sequence, followed by several specific bases that are complementary to the 3' end of a target miRNA.

2. The S-Oligo(dT) primer according to claim 1, wherein the S-Oligo(dT) primer is composed of four segments: in the 5' to 3' direction, 14 to 20 nucleotides of a universal reverse primer sequence for PCR, 14 to 20 nucleotides of a universal probe binding sequence, and 8 to 30 nucleotides of an oligo(dT) sequence, followed by 3 to 8 specific nucleotides that are complementary to the 3' end of a target miRNA.

3. The S-Oligo(dT) primer according to claim 1, wherein the S-Oligo(dT) primer is composed of four segments: in the 5' to 3' direction, 16 nucleotides of a universal reverse primer sequence for PCR, 17 nucleotides of a universal probe binding sequence, and 11 nucleotides of an oligo(dT) sequence, followed by 6 specific nucleotides that are complementary to the 3' end of a particular miRNA.

4. A method for quantitative assay of a specific miRNA using the primer according to claim 1, comprising the steps of:
S100: designing of S-Oligo(dT) Primer and miRNA Specific Forward Primer in which an S-Oligo(dT) primer and a specific forward primer for the miRNA to be assayed are designed according to the sequence information of the target miRNA;
S200: total RNA extraction in which cell lysis and total RNA extraction are performed, and the purity, integrity and concentration of the RNA sample are measured;
S300: RNA Polyadenylation in which the total RNA is polyadenylated with poly(A) polymerase to obtain a RNA-PolyA;
S400: first strand cDNA synthesis in which, using the RNA-PolyA as a template, the miRNA is then reverse transcribed into cDNA using the S-Oligo(dT) primer; and
S500: miRNA real-time PCR quantitative assay in which, using the cDNA of the miRNA as a template, a real-time PCR quantitative assay is performed using a specific forward primer and a universal reverse primer, and a data analysis is then performed on the PCR result.

5. The method according to claim 4, wherein the miRNA specific forward primer is a miRNA specific sequence without 3 to 8 nucleotides at the 3' end of the miRNA, and the miRNA universal reverse primer is a universal primer sequence having 14 to 20 nucleotides in length derived from the S-Oligo(dT) primer sequence.

6. The method according to claim 4, wherein the assay of the real-time PCR of step S500 is performed using either a fluorescent dye method or probe method, wherein the probe method uses a universal probe having 14 to 20 nucleotides in length which is derived from the S-Oligo(dT) primer sequence.

7. A method for quantitative assay of different miRNAs using the primer according to claim 1, comprising the steps of:
S100: design of S-Oligo(dT) Primer and miRNA Specific Forward Primer in which different S-Oligo(dT) primers and different specific forward primers for different miRNAs to be assayed are designed according to the sequence information of the different target miRNAs;
S200: total RNA extraction in which cell lysis and total RNA extraction are performed, and the purity, integrity and concentration of the RNA samples are measured;
S300: RNA polyadenylation in which the total RNA is polyadenylated with poly(A) polymerase to obtain a RNA-PolyA;
S400: first strand cDNA synthesis in which, using the RNA-PolyA as a template, the miRNAs are then reverse transcribed into cDNAs using a combination of the different S-Oligo(dT) primers;
S500: miRNA real-time PCR quantitative assay in which, using the cDNAs of the miRNAs as templates, a real-time PCR quantitative assay is performed using respective ones of the specific forward primers and the same universal reverse primer, and a data analysis is then performed on the PCR result.

8. The method according to claim 7, wherein the miRNA specific forward primer is a miRNA specific sequence without 3 to 8 nucleotides at the 3' end of the miRNA, and the miRNA universal reverse primer is a universal primer sequence having 14 to 20 nucleotides in length derived from the S-Oligo(dT) primer sequence.

9. The method according to claim 7, wherein the assay of the real-time PCR of step S500 is performed using either a fluorescent dye method or probe method, wherein the probe method uses a universal probe having 14 to 20 nucleotides in length which is derived from the S-Oligo(dT) primer sequence.

10. An application of the method of claim 4 or claim 7, wherein the method is applied in early diagnosis and prognosis of severe diseases.


SEQUENCE LISTING

<110> Inventors: Deming Gou, Kang Kang

<120> Primer And Method For Quantitative Assay of MicroRNA And
Application Of Same

<130> 20080502

<160> 6

<170> PatentIn version 3.5

<210> 1
<211> 24
<212> DNA
<213> Artificial Sequence

<220>
<223> Forward primer for hsa-miR-21

<400> 1
gcccgctagc ttatcagact gatg 24

<210> 2
<211> 50
<212> DNA
<213> Artificial Sequence

<220>
<223> RT primer for hsa-miR-21 in S-Oligo(dT) method

<400> 2

gtgcagggtc cgaggtcaga gccacctggg caattttttt tttttcaaca 50

<210> 3
<211> 16
<212> DNA
<213> Artificial Sequence

<220>
<223> miRNA Universal reverse primer

<400> 3
gtgcagggtc cgaggt 16

<210> 4
<211> 9
<212> DNA
<213> Artificial Sequence

<220>
<223> Universal Taqman probe 1

<400> 4
ttgcccagg 9

<210> 5
<211> 8
<212> DNA
<213> Artificial Sequence

<220>
<223> Universal Taqman probe 2

<400> 5
tggctctg 8

<210> 6
<211> 17
<212> DNA
<213> Artificial Sequence

<220>
<223> Universal Taqman probe 3

<400> 6
ttgcccaggt ggctctg 17